# EUROPÄISCHE PATENTANMELDUNG

(11) **EP 1 111 567 A2**
(43) Veröffentlichungstag der Anmeldung: **27.06.2001**
(21) Anmeldenummer: 00123591.0
(22) Anmeldetag: 28.10.2000
(51) Int. Cl.: G09B 5/00, A63B 71/06, A63B 69/00

(54) **Gerät zur Erstellung und Auswertung von Trainingsplänen**

(30) Priorität: 22.12.1999 DE 29922787 U
(71) Anmelder: Leonard, Thomas, Dr.-Ing., 58239 Schwerte (DE); Bremer, Udo, Dipl.-Ing, 59379 Selm (DE)
(72) Erfinder: Leonard, Thomas, Dr.-Ing., 58239 Schwerte (DE); Bremer, Udo, Dipl.-Ing, 59379 Selm (DE)

(57) **Zusammenfassung**

Die Erfindung betrifft ein Gerät zur Erstellung und Auswertung von Trainingsplänen, mit dem für einen oder mehrere Trainierende jeweils individuell Übungen als Bestandteil eines Trainingsprogrammes hinsichtlich ihrer Art und ihrer Eigenschaften festgelegt und in Form eines Trainingsplanes ausgegeben werden. Dabei können neben Vorgaben eines Trainers weitere Daten berücksichtigt werden, die bei zurückliegenden Übungen verfügbar wurden. Trainingsergebnisse können handschriftlich in den Trainingsplan eingetragen werden. Anschließend kann der Trainingsplan mit Hilfe der vorgeschlagenen Erfindung abgetastet und die Trainingsergebnisse gespeichert und elektronisch weiterverarbeitet werden.

## Beschreibung

Die Erfindung betrifft ein Gerät zur Erstellung und Auswertung von Trainingsplänen nach dem Oberbegriff des Schutzanspruchs 1.

Das Trainieren von körperlichen oder geistigen Fähigkeiten erfordert geeignete Übungen, die in bestimmten Abständen wiederholt werden müssen, um einen Trainingseffekt zu erzielen. Um einen optimalen Trainingseffekt zu erzielen, müssen die Übungen auf den Leistungsstand des Trainierenden angepasst sein. Da sich bei erfolgreichem Training eine Leistungssteigerung einstellen wird, ist eine ständige Anpassung des Schwierigkeitsgrades der Übungen erforderlich, um auch weiterhin optimale Trainingserfolge zu erzielen.

Durch zu schwache Trainingsreize kann keine Leistungssteigerung erzielt werden. Andererseits können zu starke Trainingsreize bei körperlichem Training im Extremfall sogar zu Gesundheitsschäden führen. Darüber hinaus wird ein unangepasstes Training auf Dauer den Trainierenden demotivieren, da zu schwache Trainingsreize auf der einen Seite nicht zu dem erhofften Trainingserfolg führen werden und zu hohe Leistungsanforderungen andererseits Frustration verursachen werden.

Der Schwierigkeitsgrad des Trainings muss kontinuierlich dem Leistungsstand des Trainierenden angepasst werden. Diese Anpassung kann auf der Grundlage von Trainingsdaten erfolgen. Zu diesen Trainingsdaten zählen zunächst objektive Größen, die bereits vor einer Übung bekannt sind (a priori Größen), wie beispielsweise das Gewicht einer Hantel oder die Länge einer zurückzulegenden Strecke, ferner solche objektiven Größen, die während der Übung ggf. durch die Verwendung von weiteren Hilfsmitteln bestimmt werden (a posteriori Größen), z.B. die Zeit, in der eine bestimmte Strecke zurückgelegt wurde, und schließlich subjektive Größen, also Einschätzungen des Trainierenden oder des Trainers.

Die a posteriori Größen können ggf. durch elektronische Messeinrichtungen bestimmt und anschließend ohne Medienbruch einer elektronischen Weiterverarbeitung zur Verfügung gestellt werden (WO 9840126 A1 19980917). Sofern ein Training aus vielen verschiedenen Übungen besteht, wäre es konsequent, bei jeder dieser Übungen in entsprechender Weise Messwerte zu erfassen. Dies ist jedoch offensichtlich mit hohen Kosten verbunden. Darüber hinaus ist die elektronische Bestimmung von a posteriori Größen bei bestimmten Übungen nicht sinnvoll. Bei Kraftübungen mit Hanteln steht das Gewicht der Hanteln beispielsweise als a priori Größe zur Verfügung. Dennoch reicht das Hantelgewicht für eine Einschätzung des Leistungsstandes des Trainierenden in Bezug auf diese Übung allein nicht aus. Auch zwei Trainierende mit unterschiedlicher Leistungsfähigkeit könnten die Hantel erfolgreich stemmen, wobei sie die Übung jedoch als unterschiedlich schwer empfinden würden. Neben der a priori Größe ist daher auch eine subjektive Bewertung des Schwierigkeitsgrades der Übung erforderlich.

Vielfach können Übungen leicht so konzipiert werden, dass, anstatt mit hohem Aufwand eine a posteriori Größe zu bestimmen, eine oder mehrere a priori Größen für eine Übung vorgegeben werden und nach Beendigung der Übung eine subjektive Bewertung durch den Trainierenden selbst oder durch einen Trainer erfolgt

In die subjektive Bewertung des Trainierenden gehen indirekt auch schwer erfassbare Größen wie seine Motivation oder Belastbarkeit ein. Sofern diese Größen bei der Bemessung des weiteren Trainings geeignet berücksichtigt werden, können Misserfolgserlebnisse leichter vermieden werden, so dass langfristig die Freude am Training erhalten bleibt. Insbesondere bei kommerziell angebotenen Trainingsdienstleistungen im Freizeitbereich kommt diesem Aspekt große Bedeutung zu.

Die Konzeption eines Trainingsplanes und die Bemessung der einzelnen Übungen hinsichtlich ihrer Art und ihres Schwierigkeitsgrades fällt prinzipiell in den Aufgabenbereich eines Trainers. Da gerade bei einem Training, das als Dienstleistung im Freizeitbereich angeboten wird, der Trainer mitunter eine Vielzahl von Trainierenden zu betreuen hat, ist eine individuelle und kontinuierliche Anpassung des Trainingsplanes an den Leistungsstand der Trainierenden nur schwer zu erreichen.

Zur Unterstützung des Trainers bei der Konzeption des Trainingsplanes kann prinzipiell eine Datenverarbeitungsanlage eingesetzt werden. Sinnvollerweise müssen dabei neben trainingsphysiologischen und trainingspsychologischen Gesichtspunkten auch die verfügbaren Trainingsdaten berücksichtigt werden. Insbesondere bei einem Training, das als Dienstleistung zur Freizeitgestaltung angeboten wird, kann den Trainierenden jedoch nicht zugemutet werden, sich mit den Bedienungsabläufen einer Datenerfassungsanlage auseinandersetzen zu müssen.

Der in Schutzanspruch 1 angegebenen Erfindung liegt das Problem zu Grunde, eine Einrichtung zu schaffen, mit der für mindestens einen Trainierenden mindestens eine Übung hinsichtlich ihrer Art und ihrer Eigenschaften festgelegt und in Form eines Trainingsplanes ausgegeben wird, wobei neben Vorgaben eines Trainers weitere Daten berücksichtigt werden können, die bei zurückliegenden Übungen verfügbar wurden.

Dieses Problem wird durch ein Gerät nach den Merkmalen des Schutzanspruchs 1 gelöst.

Mit der Erfindung wird erreicht, dass Trainingsdaten auf effiziente Weise elektronisch erfasst und in einer Datenbank gespeichert werden können, ohne dass eine unbequeme und möglicherweise komplizierte manuelle Eingabe erforderlich wäre. Auf der Grundlage dieser Trainingsdaten wird anschließend unter Berücksichtigung trainingsphysiologischer und trainingspsychologischer Gesichtspunkte, sowie Vorgaben eines Trainers, automatisch ein Vorschlag für zukünftige Übungen erstellt und in Form eines Trainingsplanes ausgegeben. Auf diese Weise wird ein beaufsichtigender Trainer bei der Konzeption und der Anpassung des Trainings an den Leistungsstand der Trainierenden optimal unterstützt.

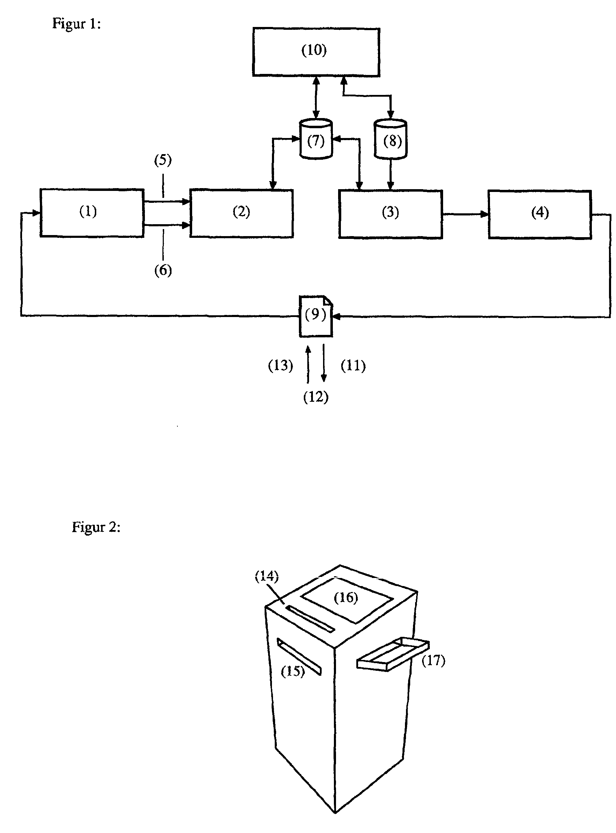

Eine vorteilhafte Ausgestaltung der Erfindung ist in Schutzanspruch 2 angegeben und wird mit Hilfe von Figur 1 erläutert. Die Weiterbildung nach Schutzanspruch 2 ermöglicht die Anzeige oder die graphische Darstellung auf einer Visualisierungs- und Eingabeeinrichtung (10) von in der Datenbank (7) abgespeicherten Trainingsdaten eines oder mehrerer Trainierender und der in Datenbank (8) abgespeicherten Trainingsvorgaben und Trainingsregeln, sowie deren manuelle Veränderung und Ergänzung durch einen Trainierenden oder Trainer.

Ein Ausführungsbeispiel der Erfindung wird anhand der Figuren 1 und 2 erläutert. Durch eine Öffnung im Gehäuse (14) des Gerätes werden Trainingspläne (9) aus Papier oder Pappe mit bereits ausgeführten Übungen eingeführt. Diese Trainingspläne (9) enthalten Trainingsanweisungen (11) für den Trainierenden oder Trainer (12). In den Trainingsplan wurden zuvor handschriftliche Eintragungen (13) vorgenommen oder Markierungen auf gedruckten Skalen angebracht, die Rückschlüsse auf den Leistungsstand des Trainierenden erlauben. Mit Hilfe einer optoelektronischen Einleseeinrichtung (1) werden maschinell erzeugte Markierungen auf dem Trainingsplan, welche den Trainierenden und den vorliegenden Trainingsplan eindeutig identifizieren (5), sowie die handschriftlichen Eintragungen (6) abgetastet und an eine Einrichtung zur Auswertung dieser Markierungen (2) weitergeleitet. Der Trainingsplan wird anschließend durch eine Öffnung im Gehäuse des Gerätes (15) wieder nach außen geführt. Die maschinellen Markierungen (5) und handschriftlichen Eintragungen (6) werden in der Auswertungseinrichtung (2) interpretiert und ausgewertet und in einer Datenbank (7) abgespeichert. Die Datenbank (7) kann sowohl in das Gerät integriert, als auch Bestandteil einer zusätzlichen externen Datenverarbeitungsanlage sein, welche über eine geeignete Datenverbindung an das Gerät angeschlossen ist. Mit Hilfe einer Einrichtung zur Visualisierung und Dateneingabe (10) können die in der Datenbank (7) gespeicherten Informationen dargestellt und verändert werden. Die Darstellung dieser Informationen kann in graphisch aufbereiteter Form erfolgen. Zusätzlich erlaubt die Einrichtung zur Visualisierung und Dateneingabe (10) die Erstellung und Veränderung von Vorgaben für die weitere Ausgestaltung des Trainings. Diese Vorgaben werden in einer weiteren Datenbank (8), welche wiederum in das Gerät integriert oder separat ausgeführt sein kann, abgespeichert. Die Einrichtung zur Visualisierung und Dateneingabe (10) kann durch einen berührungsempfindlichen Bildschirm (16) ausgeführt werden, welcher in das Gerät integriert ist. Auf der Basis der in den Datenbanken (7) und (8) gespeicherten Daten wird von einer Einrichtung zur Konzeption von Trainingsplänen (3) ein neuer Trainingsplan zusammengestellt und anschließend mit Hilfe einer Ausgabeeinrichtung (4) ausgegeben. Diese Ausgabeeinrichtung kann durch ein in das Gerät integriertes Druckwerk ausgeführt werden, welches den neuen Trainingsplan auf Papier oder Karton ausgibt und in der Auffangvorrichtung (17) abgelegt.

Durch das in der Erfindung beschriebene Gerät kann für einen Trainierenden maschinell ein ständig an seinen Leistungsstand angepasster Trainingsplan erstellt werden. Dabei können die bei zurückliegenden Übungen erzielten Ergebnisse geeignet berücksichtigt werden, ohne dass diese zuvor von dem Trainierenden oder einer dritten Person manuell in das Gerät eingegeben werden müssten. Für den Trainierenden beschränken sich die notwendigen Bedienungsabläufe auf einfache und im allgemeinen aus dem Alltag vertraute Handhabungen, wie das Lesen von Anweisungen und das handschriftliche Ausfüllen von Formularen.

## Patentansprüche

1. Gerät zur Erstellung und Auswertung von Trainingsplänen,
dadurch gekennzeichnet, dass das Gerät aus
• mindestens einer Einrichtung zum Einlesen (1) von Trainingsplänen,
• einer Einrichtung zur Auswertung (2) der eingelesenen Trainingspläne,
• einer Einrichtung zur Konzeption (3) von Trainingsplänen,
• und mindestens einer Einrichtung zur Ausgabe (4) von Trainingsplänen
besteht.

2. Gerät zur Erstellung und Auswertung von Trainingsplänen nach Schutzanspruch 1,
dadurch gekennzeichnet, dass
• das Gerät über mindestens einen Monitor verfügt, mit dem Trainingsvorgaben und Trainingsregeln angezeigt werden, und
• das Gerät über mindestens eine Einrichtung zur manuellen Eingabe verfügt, mit der Trainingsvorgaben und Trainingsregeln verändert oder ergänzt werden können.
